(19) Europäisches Patentamt
European Patent Office
Office européen des brevets

(11) **EP 2 471 557 A1**

(12) **EUROPEAN PATENT APPLICATION**

(43) Date of publication:
**04.07.2012 Bulletin 2012/27**

(51) Int Cl.:
*A61K 51/08* (2006.01)   *A61P 35/00* (2006.01)

(21) Application number: **10197071.3**

(22) Date of filing: **27.12.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Università degli Studi di Genova**
  **16126 Genova (IT)**
- **Instituto Europeo di Oncologia S.r.l.**
  **20121 Milano (IT)**

(72) Inventors:
- **Caviglioli, Gabriele**
  **16147 Genova (IT)**

- **Parodi, Brunella**
  **16167 Genova (IT)**
- **Cafaggi, Sergio**
  **16121 Genova (IT)**
- **Russo, Eleonora**
  **16147 Genova (IT)**
- **Cirrincione Paola**
  **16148 Genova (IT)**
- **Chinol, Marco**
  **20141 Milano (IT)**
- **Paganelli, Giovanni**
  **20141 Milano (IT)**

(74) Representative: **Ferreccio, Rinaldo**
  **Botti & Ferrari S.r.l.**
  **Via Cappellini, 11**
  **20124 Milano (IT)**

(54) **A conjugate of human albumin and 2-(4-isothiocyanatobenzyl)-1,4,7,10-tetraazacyclododecane-1,4,7,10-tetraacetic acid useful for the localization of radionuclides for diagnostic and therapeutic purposes**

(57)    A conjugate of human albumin (HA) and 2-(4-isothiocyanatobenzyl)-1,4,7,10-tetraazacyclododecane-1,4,7,10-tetraacetic acid (p-SCN-Bn-DOTA) insoluble in aqueous medium at a pH of 3-8.5 and whose IR spectrum shows an absorption at about 700 cm$^{-1}$ is described; a complex is further described consisting of a conjugate of HA and p-SCN-Bn-DOTA (HAC) according to claim 1 or 2 and a radioactive isotope of an element selected among the group consisting of P, Sr, Rh, Pd, I, Sm, Er, Au, At, Bi, In, Lu, Y, Re, Cu and Ag, as well as procedures for preparing said conjugate and said complex as well as the use of such conjugate in the identification of mammalian neoplastic lesions by the R.O.L.L. methodology ("Radioguided Occult Lesion Localization").

Fig. 3

EP 2 471 557 A1

**Description**

Field of application

The present invention is directed to the field of pharmaceutical and diagnostic industry and refers to a microdispersed system insoluble in aqueous environment, for the localization of radionuclides for diagnostic and therapeutic purposes and to a process for its preparation.

More particularly, the invention refers to a conjugate of human albumin (HA) and 2-(4-isothiocyanatobenzyl)-1,4,7,10-tetraazacyclododecane-1,4,7,10-tetraacetic acid (p-SCN-Bn-DOTA) insoluble in aqueous environment at pH values within 3 and 8.5, able to complex radionuclides, useful for diagnostic and therapeutic purposes, in particular for the treatment of mammalian neoplasias.

Known art

Over the last 20 years, the identification of mammalian neoplastic lesions at an earlier and earlier stage has allowed the development of more and more conservative surgical treatments.

Until few years ago the diagnosis of a non-invasive mammalian carcinoma did not exceed the 5% of cases. In the last decade the diffusion on a large scale of instrumental investigations, in particular of mammography and ultrasound, has allowed an increase in the identification of clinically hidden tumours (not perceptible by touch at the time of physical examination), which are histologically non-invasive in a percentage close to 25-30%. The objective of obtaining a precise pre-surgery localization of the lesion and a complete surgical removal has been the stimulus that has led researchers of the Istituto Europeo di Oncologia to develop a new technique for the localization and surgical treatment of nonpalpable mammalian lesions, which combines the use of radioactive tracers with the use of the probe for radioguided surgery (Paganelli G., Luini A., Veronesi U. et al. 2000. Use of technetium-99m-labeled colloid albumin for preoperative and intraoperative localization of nonpalpable breast lesions. Journal of the American College of Surgeons 190(6):692-699; Paganelli G., Veronesi U. 2002. Innovation in early breast cancer surgery: radio-guided occult lesion localization and sentinel node biopsy. Nucl Med Commun 23(7):625-627).

This methodology for the localization of nonpalpable mammalian lesions, called R.O.L.L., English acronym for "Radioguided Occult Lesion Localization", includes the introduction of a suspension of macroaggregates of human albumin labelled with radioactive technetium ($^{99m}$Tc) into a group of microcalcifications or a small clinically nonpalpable nodule. By means of the probe for radioguided surgery, these lesions, frequently of tumoral nature, can be surgically removed in a targeted way maintaining the integrity of the breast.

The procedure of inoculation includes the use of macroaggregates of human albumin (MAA) of variable 10-100 $\mu$m diameter, labelled with $^{99m}$Tc with a specific activity of about 1700 MBq/mg. The day preceding the surgical procedure 10-15 MBq of radiolabelled drug are suspended in 0.2 ml of saline and injected, guided by ultrasound, into the centre of an opacity or, guided by the mammographic stereotaxic system, of a small group of microcalcifications. The verification of the correct localization can be obtained introducing into the lesion, immediately after the radioactive substance, a minimum amount of radiopaque solution. For a further verification, the so treated patient undergoes a scintigraphic investigation of the breast. The overlay of the image acquired by the gamma chamber, properly enlarged, and the mammographic one allows to confirm that the site of injection exactly corresponds to the site of the lesion.

The validity of this methodology is demonstrated by its diffusion also shown in various scientific reports (van der Ploeg C. et al. 2008. 'Radioguided occult lesion localisation' (ROLL) for nonpalpable breast lesions: A review of the relevant literature. EJSO 34(1):1-5; Povoski P., et al. 2009. A comprehensive overview of radioguided surgery using gamma detection probe technology. World J Surg Oncol 7).

The currently used radiolabelled drug (Technetium-MAA) does not allow, due to the short half-life of the radioactive decay ($t_{1/2}$= 6.02 hours) of the 99 radioisotope of technetium ($^{99m}$Tc), a planning of surgical procedures able to address the growing request of patients with lesions that should undergo radioisotopic localization.

Hence, the use of a radiolabelled drug with a longer half-life would allow to plan surgical procedures on several days and, not negligible aspect, also to carry out the localization as an outpatient exam as well as its verifications in the days preceding surgery, further reducing hospitalization times and costs.

The radiolabelled drug used up to date is an aspecific complex between macroaggregates of human albumin (MAA) and $^{99m}$Tc that is prepared at the time of use through different marketed kits from various manufacturers: Bracco, CIS-US, Draximage, GE Healthcare and Mallinckrodt. These kits differentiate for the amount of aggregated human albumin, SnCl$_2$, and preservatives they contain. They are provided to the centres of nuclear medicine as freeze-dried powders of the non-radioactive ingredients, preserved under a nitrogen atmosphere. The operator adds 40-100 mCi of sodium pertechnetate (Na$^{99m}$TcO$_4$) to obtain the final radiolabelled drug. After stabilization at room temperature for 15 min, in order to ensure the maximum labelling of $^{99m}$Tc to human albumin (HA), the kit can be diluted with isotonic sterile solution of 0.9% NaCl (w/v) (or solution of sodium chloride for parenteral preparations (FU XII), hereinafter 0.9% NaCl) and injected into the patient.

The soluble form of $^{99m}$Tc, i.e. sodium pertechnetate (Na$^{99m}$TcO$_4$), is obtained from $^{99}$Mo/$^{99m}$Tc generators, by elution with saline.

The operator adds to the freeze-dried MAA a volume of few milliliters of Na$^{99m}$TcO$_4$ in injectable saline, depending on the radioactivity that is intended to achieve.

SnCl$_2$ is a strong reducing agent contained in MAAs themselves that, put in contact with the weak oxidizing agent Na$^{99m}$TcO$_4$, allows complete conversion of the soluble form of $^{99m}$Tc into the Tc$^{+3}$ ion that precipitates as TcCl$_3$ within the MAAs. The strength and amount of the reducing agent are such to ensure complete reduction of Na$^{99m}$TcO$_4$ avoiding the need to remove residual Na$^{99m}$TcO$_4$.

Sn(II) must be converted into the water-soluble SnCl$_4$ but, if conditions are not carefully controlled, it can convert, in aqueous environment and in the presence of oxygen, also in Sn(OH)Cl, poorly soluble in aqueous environment. The ATC code for this radiolabelled drug is V09EB01 (Technetium particles for injection (Macrosalb)).

A typical formulation of freeze-dried MAAs (MAASOL GE Healthcare) contains:

1.75 mg MAA/vial
Stannous Chloride dihydrate 0.175 mg/vial
Sodium acetate
Poloxamer 238
N$_2$ atmosphere

Sodium acetate in solution will form the buffering system at pH 4.8-5.8, pH at which MAAs form and stabilize. Poloxamer is an adjuvant that aids the redispersion of the freeze-dried product in the injection medium keeping the size of the aggregates smaller than 100 μm. The nitrogen atmosphere is necessary to prevent the oxidation of stannous chloride by atmospheric oxygen.

The main diagnostic indication for which MAAs are licensed after labelling with $^{99m}$Tc is pulmonary perfusion scintigraphy, as a secondary indication they are used in venous scintigraphy and in some cases they are also indicated for the evaluation of the peritoneal-venous shunt after intraperitoneal administration.

Additionally, it should be reminded that the Italian Drug Agency (AIFA) recently released an alert [Information Note of March 2009 concerning radiolabelled drugs containing albumin (Maasol, Macrotec, Nanocoll, Pulmocis, Technescan Lyomaa, Ventricoll): safety information.] concerning the reporting of anaphylactic adverse reactions with lethal outcome associated to intravascular administration of radiolabelled drugs containing HA.

Since a causal relationship between the adverse reaction and MAA could not be established with certainty, it is legitimate to suspect also other components of the preparation, not least the Sn compounds.

The aspecificity of the association between Tc and MAA could also be the cause of the phenomenon, however recorded as not very frequent, of diffusion and/or drainage of the radionuclide in healthy tissues and/or in the lymphatic circulation.

These latter considerations, associated with the primary need of finding a radiolabelled drug with a half-life longer than the 6.02 hours of $^{99m}$Tc, represent the rationale that has led to the achievement of the invention disclosed herein.

The macroaggregates of HA (MAA) are obtained through different procedures that differentiate for some details and have given rise to as many patents.

In 1969 Bowen et al. (Preparation of Technetium-99m Human Serum Albumin Macroaggregates, Am J of Hospital Pharm., 26:529-534, 1969) had already provided a detailed description of these macroaggregates.

When referring to MAA one should refer to particles of HA insoluble in aqueous phase, with a range of dimensional distribution between 3 and 200 μm that are reduced to 10 to 100 μm for therapeutic use. The selection of the particle size is carried out by filtrations through controlled porosity membranes, to exclude particles with larger sizes, and by centrifugation in order to remove particles with smaller sizes not readily sedimented.

The various procedures for the preparation of MAAs include:

1. denaturation of HA by exposure to extreme pHs, for example pH 1-2 by using strong acids (sulphuric acid, hydrochloric acid), to heat, bases or organic solvents;
2. addition of a suitable amount of SnCl$_2$ in solution stabilized with HCl;
3. adjustment of pH in the range between 4 and 6, generally with an acetate buffer, in the proximity of the isoelectric point of denaturated HA (about 5.3), that determines an initial precipitation of colloidal aggregates;
4. subsequent heating between 50 and 90°C, but in some cases 110°C are also reached, that determines the formation of MAAs and their stabilization. These heating modalities, which influence the dimensional distribution of the aggregates, are obtained in the most different ways also alterning cycles of heating and quick cooling.

MAAs, dimensionally selected by filtration and centrifugation, can then be freeze-dried with the addition of suitable adjuvants: anti-aggregating, antifoam agents, preservatives, or not denatured HA to facilitate reconstitution.

Sterility and apyrogenicity of the final product is guaranteed by the sterility and apyrogenicity of the used materials and by the control of environmental conditions (US 4,024,233; US 4,094,965).

In a recent patent (US 6,730,286), inter alia, an improvement in the production of these MAAs through preventive purification of the solution of HA by ultrafiltration is underlined.

From the analysis of preceding patents and publications it is clear that the formation of MAAs is a process requiring several preparative steps. The direct labelling of MAA with $^{111}$In, as it has been demonstrated by Watanabe N. et al. (Journal of Nuclear Medicine 38(10):1590-1592, 1997), produced a stability of the MAA-radioisotope complex as short as 180 minutes.

This result suggested to conjugate to MAAs complexes able to bind radioisotopes for a longer time.

Then, at first the Applicants investigated the possibility to carry out a conjugation reaction with p-SCN-Bn-DOTA directly on MAAs. Such reaction produced poor results in terms of conjugation and of reproducibility. In fact, the formation of MAAs is based on the interactions between the protein amine and carboxylic groups, and free amine groups are present on the surface of MAAs in minimal and variable amounts or they are even absent.

As reported by Patil (Drug Dev. Res. 58:219-247, 2003) HA was also transformed into micro- and nanospheres that could be used for diagnostic and therapeutic purposes. It is clear that the microspheres (MS) can be an interesting substrate for preparing a carrier for radiolabelled drugs.

The formation of microspheres involves complex multi-step procedures, and the use of oily phases, organic solvents, and reticulating agents such as glutaraldehyde. For example, a preparative modality involves emulsifying an HA aqueous solution in oil (e.g. cottonseed oil), the emulsion W/O is heated to 110°C or more until microspheres are formed. MS are separated by filtration and washed with solvents (e.g. acetone, ether, heptane).

Another preparative mode involves pouring an aqueous solution of HA in anhydrous ethanol at -15°C. Microspheres are then hardened with glutaraldehyde. The reaction is quenched with metabisulfite. Eventually microspheres are filtered and washed with abundant water.

It is to consider anyway that the architecture of MS involves an interaction between carboxylic and amine groups of the protein, or the consumption of these amine groups for the cross-linking reaction with a reticulating agent such as glutaraldehyde.

On the basis of these considerations it is clear that the formation of microspheres of HA (MSHA) involves a complex preparation with several preparative steps characterized by the wide use of non aqueous solvents.

Schiller et al. (Nuclear Medicine and Biology 35(2):227-232, 2008) studied the labelling with $^{86}$Y of microspheres of HA conjugated with DOTA. Such conjugation was obtained using p-SCN-Bn-DOTA. Such microspheres designed for the treatment of liver neoplasias were assayed by injecting, in the caudal vein of Wistar rats, 50-100 $\mu$g of MS (2 MBq). Microspheres having a diameter between 20 and 30 $\mu$m were obtained by heating (100-130°C) an aqueous solution of HA emulsified with olive oil. MS had a specific surface of 0.26 m$^2$/g and a conjugation ratio of $2\times10^{-7}$ moles per milligram of microspheres. Conjugation occurred using a p-SCN-Bn-DOTA:MSHA molar ratio of 100:1 in aqueous phase at pH 9 using $Na_2B_4O_7$ as a buffer.

Using the same reaction conditions but using HA as a substrate, conjugation does not occur, probably because the protein undergoes partial hydrolysis due to the excessively high pH.

Jakubowski et al. (J. Anal. At Spectrom. 23(11):1497-1507, 2008) describe a conjugation procedure of bovine serum albumin (BSA) by p-SCN-Bn-DOTA in carbonate buffer at pH 9 at 20°C, using 6 nmol of BSA, an optimized reaction molar ratio p-SCN-Bn-DOTA/BSA of 40/1 and a conjugation reaction time between 16 and 24 hours. The conjugation product DOTA-BSA obtained by these authors is soluble in the aqueous medium of reaction. In fact, the solubility of their product in acetate buffer (pH 7-7.5; 0.5M) also allows the elution of the conjugate in the subsequent chromatographic purification procedure described by the authors. Always in aqueous solution and in the presence of acetate buffer, i.e. in homogeneous phase, the conjugation product is labelled with Europium, with a reaction molar ratio of Eu/p-SCN-Bn-DOTA of 10/1, using a labelling temperature of 37°C for 30 min. The maximum conjugation molar ratio, estimated by measuring the TXRF (total reflection X-ray fluorescence) of Eu in the protein, conjugated and labelled, is 4.1 Eu/BSA.

Summary of the invention

In one aspect, the present invention relates to a conjugate of human albumin or a water-soluble derivative or analogue thereof (HA) and 2-(4-isothiocyanatobenzyl)-1,4,7,10-tetraazacyclododecane-1,4,7,10-tetraacetic acid (p-SCN-Bn-DOTA) insoluble in aqueous medium at a pH of 3-8.5 and whose IR spectrum shows an absorption at about 700 cm$^{-1}$.

HA, as intended hereinafter, means human albumin or a water-soluble derivative or analogue thereof.

In another aspect, the present invention relates to a complex consisting of said conjugate of HA and p-SCN-Bn-DOTA (HAC) and of a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu. In particular the radioactive isotope is selected among the group consisting of $^{111}$In, $^{177}$Lu, $^{90}$Y, $^{67}$Cu and radioactive lanthanides and is preferably [177]Lu.

In particular, among the radioactive lanthanides, $^{140}$La, $^{149}$Pm, $^{153}$Sm, $^{152}$Tb, $^{166}$Ho, 169Er and $^{175}$Yb are cited.

The above mentioned complex can be used for in vivo localization of radionuclides in organs or tissues of the human body for diagnostic or therapeutic purpose and in particular for the localization of mammalian neoplastic lesions by the R.O.L.L. methodology ("Radio guided Occult Lesion Localization").

According to another aspect, the invention relates to a process for preparing a conjugate of HA and 2-(4-isothiocyanatobenzyl)-1,4,7,10-tetraazacyclododecane-1,4,7,10-tetraacetic acid (p-SCN-Bn-DOTA), comprising the steps of:

a) reacting HA with p-SCN-Bn-DOTA in aqueous medium at a temperature between 35°C and 45°C, with the formation of a suspension, containing a solid phase suspended in a liquid phase;

b) separating said solid phase, containing said conjugate, from said liquid phase, possibly containing unreacted p-SCN-Bn-DOTA.

Said separation step b) can be a filtration step or, preferably, an ultrafiltration step.

The solid obtained from the filtration step, as well as the retentate from the ultrafiltration step, can be subjected to freeze-drying.

The reaction between HA and p-SCN-Bn-DOTA is carried out preferably at a temperature of 38-42°C for 8-24 hours, preferably for 10-16 hours. The aqueous medium in which said reaction occurs is preferably an aqueous solution with ionic strength equal to 0.1-0.5M, in particular a 0.9% NaCl solution, optionally buffered at pH 4-8.5.

In the ultrafiltration step a membrane having a cut-off of 10 kDa is generally used.

In the reaction between p-SCN-Bn-DOTA and HA, p-SCN-Bn-DOTA and HA are typically used in a molar ratio between 100:1 and 1:1, preferably between 30:1 1 and 16:1.

Preferably HA subjected to the reaction step a) with p-SCN-Bn-DOTA is previously purified by ultrafiltration or dialysis with a membrane having a cut-off of 10 kDa, in the presence of a solution of ionic strength 0.1-0.5M, in order to remove the thermal stabilizers present in commercially available HA, in particular sodium caprylate and sodium acetyl tryptophanate.

In a further aspect, the present invention refers to a conjugate of human albumin or a water-soluble derivative or analogue thereof (HA) and 2-(4-isothiocyanatobenzyl)-1,4,7,10-tetraazacyclododecane-1,4,7,10-tetraacetic acid (p-SCN-Bn-DOTA) insoluble in aqueous medium at a pH of 3-8.5 and whose IR spectrum shows an absorption at about 700 cm$^{-1}$, obtainable by the above described process.

In a further aspect, the present invention refers to a process for preparing a complex as described above, comprising the steps of:

a) dispersing a conjugate as described above in aqueous buffer at a pH of 4-6;

b) adding a water-soluble compound containing an ion of a radioactive isotope of an element selected among the group consisting of Sr, Rh, Pd,

Sm, Er, Au, Bi, In, Lu, Y, Ce, Pr, Nd, Pm, Sa, Eu, Gd, Tb, Dy, Ho, Tm, Yb, Ga, Ni, Co, Fe and Cu and heating under stirring at a temperature of 30-90°C for 20-40 minutes.

The water-soluble compound added to the dispersion from step a) is, in a particular aspect of the present invention, [177]LuCl$_3$.

As an aqueous buffer in step b) acetic acid/acetate buffer is preferably used, in particular 1M with a pH of 5.

In a further aspect, the present invention refers to a kit for preparing a complex as described above, including a conjugate of HA and p-SCN-Bn-DOTA and a water-soluble compound containing an ion of a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu.

Said water-soluble compound is preferably [177]LuCl$_3$.

In the kit according to the invention, said conjugate is conveniently in a lyophilized form and said water-soluble compound is in the form of an aqueous solution.

The conjugate according to the present invention allows to obtain complexes with stable radioisotopes, suitable in particular for the localization of mammalian neoplastic lesions, in particular with the R.O.L.L. methodology. The above mentioned complexes, insoluble in aqueous medium, are not subjected to diffusion or drainage phenomena of the radionuclide in healthy tissues and/or in the lymphatic circulation and are instead provided of a half-life that allows to plan possible surgical procedures, following the diagnostic results, over several days.

Further advantages and characteristics of the present invention will result from the following detailed description, in which reference is made to some figures, as described hereinafter.

Short description of the figures

Figure 1 shows the variation of the degree of substitution (DS) of p-SCN-Bn-DOTA in the HAC conjugate upon varying the concentration of carbonate buffer.

Figure 2 shows a comparison between the gelatinous precipitate obtained by the process according to the present invention (right microtube) and the solution deriving from treatment of HAP with carbonate buffer and DOTA for 20 h at 40°C (left microtube).

Figure 2A shows the formation of a gelatinous precipitate in the first hour of reaction of the process according to the invention.

Figure 3 shows a comparison between the IR spectrum of the freeze-dried conjugate according to the invention (top) and the IR spectrum of purified albumin (HAP) (bottom) subjected to the same reaction conditions used in the preparation of the conjugate according to the invention, using DOTA instead of p-SCN-Bn-DOTA.

Figure 4 is a picture displaying a comparison between the freeze-dried conjugate according to the invention (indicated with C) and purified freeze-dried albumin (HAP).

Figure 5 shows a comparison between DSC profiles of the conjugate according to the invention (full line) and HAP (broken line).

Figure 5A shows DSC profiles of a freeze-dried sample of purified albumin (HAP) pre-treated at 100°C (full line) and subjected to a subsequent gradient from -20°C to 250°C (broken line).

Figure 5B shows DSC profiles of a freeze-dried sample of a conjugate according to the invention pre-treated at 100°C (full line) and subjected to subsequent gradient from -20°C to 250°C (broken line).

Figure 6 is a picture displaying a comparison between residual samples from the DSC study of Figure 5, wherein the residue of the conjugate according to the invention is the one on the right side.

Figure 7 is a micrograph of a sample of a conjugate according to the present invention in colloidal suspension in 1M sodium acetate pH 5 subjected to prolonged (20 h) heating (40°C).

Figure 8 is a graph reporting a calibration curve (absorbance vs. $\mu$moles) built on the basis of the data reported in the Table 3 of Example 1.

Figure 9 is a graph reporting a calibration curve (emission at 261.542 nm vs. $\mu$g/ml) built on the basis of data reported in the Table 5 of Example 2.

Figure 10 displays an ITLC plate (instant thin-layer chromatography) carried out on HAC samples radiolabelled with $^{111}$In (1 mCi/mg), in order to evaluate its radiochemical purity.

Figure 11 displays an ITLC plate (instant thin-layer chromatography) carried out on HAC samples radiolabelled with $^{177}$Lu (1 mCi/mg), in order to evaluate its radiochemical purity.

Figure 12 displays an ITLC plate (instant thin-layer chromatography) carried out on HAC samples radiolabelled with $^{177}$Lu (2 mCi/mg) from another batch, in order to evaluate its radiochemical purity.

Detailed description

The present invention relates to a conjugate of HA and p-SCN-Bn-DOTA, hereinafter called HAC, insoluble in aqueous environment between pH 3 and pH 8.5, able to complex radionuclides, obtainable with a procedure including a single reactive step. The above mentioned conjugate represents an insoluble microdispersed system that can substitute MAAs because it can complex by coordination some radionuclides that can be used for diagnostic and therapeutic purposes. In fact, the conjugate according to the invention is able to stably complex various ions and thus can be labelled with different radioisotopes forming complexes with DOTA that are stable and kinetically inert.

DOTA, or 1,4,7,10-tetraazaciclododecane-1,4,7,10-tetraacetic acid, is a known macrocyclic ring chelating agent, giving complexes stable in time with various metals, with a two-step kinetics of incorporation of the metal in the macrocycle, consisting in the subsequent deprotonation of nitrogen atoms and carboxylic groups of the ring. This mechanism has a first pH-controlled quick step that involves only the nitrogen atoms on the ring, and a second slower step (step of cage closure), in which participate the carboxylic groups that give stability to the complex.

The conjugate according to the present invention contains the DOTA structural moiety, introduced in the HA molecule thanks to the reactivity of the isothiocyanate function of p-SCN-Bn-DOTA.

The degree of conjugation of p-SCN-Bn-DOTA in HAC is such to allow a suitable radioisotopic labelling for its application in the R.O.L.L. or in other similar diagnostic and therapeutic methodologies. Furthermore, differently from other protein substrates, such product resists to the labelling procedures that involve high temperatures, for example 90°C for 30 min, conditions necessary to effectively complex, in the DOTA macrocycle, lanthanides such as lutetium.

HAC is able to be inoculated in tissues, following radioisotopic labelling, as a colloidal precipitate or as a microdispersion, also derived from the reconstitution of freeze-dried samples, and there to exert its diagnostic and therapeutic activity for a time not shorter than the half-life of the radioactive decay of the radionuclide.

Among the useful radionuclides in the achievement of the present invention some gamma emitters can be cited, such as $^{111}$In, with a $t_{1/2}$ of 67 h (2.79 days) and $^{177}$Lu, with $t_{1/2}$ of 6.64 days. The latter is particularly interesting because it displays, beside the gamma emission necessary for imaging, a $\beta^-$ emission of intermediate energy (500 keV, maximum penetration 2 mm) very useful in the treatment of small size tumors. Furthermore, the energy of gamma photons emitted from $^{177}$Lu is very close to that of $^{99m}$Tc, the most widely used radioisotope in nuclear medicine, and then compatible with the currently used $\gamma$-chambers.

Among the other radionuclides that can be used in the present invention $^{90}$Y, and $^{67}$Cu ($t_{1/2}$ 2.58 days) can be cited. In particular, $^{90}$Y is considered by many the most important isotope in nuclear-medical therapy. Such radionuclide is a pure high energy $\beta^-$ (2.27 kEv) with maximum penetration in tissues exceeding 1 cm and $t_{1/2}$ of 64 hours (2.6 days). Since it lacks $\gamma$ emissions, it cannot be used in imaging or in dosimetry, though the great advantage of this radionuclide is that also the tumour cells more distant from the inoculation point, lying in a radius of 2 cm, can be hit; this effect, known as "crossfire effect", is considered important in the treatment of solid tumours.

Further directions about useful radionuclides for the present invention can be taken from "Radionuclide selection and model absorbed dose calculations for radiolabelled tumour associated antibodies" B. Wessels, R. Rogus. Med Phys. 11 (5) 1984 pages 638-645, and from "Metallic radionuclides for radioimmunotherapy." A.R. Fritzberg, C.F. Meares in "Cancer radioimmunotherapy: Present and Future" Harwood Academic Publishers, Switzerland, Editore Riva P, pages 57-79, 1999.

In the following Table 1 a list is reported by way of indication, and not exhaustive, of the radionuclides that can be used in the present invention.

Table 1

| Radionuclide | $t_{1/2}$ | Decay (MeV) | Rmax |
|---|---|---|---|
| 67Cu | 2.58 d | $\beta(0.54)$, $\gamma(0.185)$ | 1.8 mm |
| 64Cu | 12.8 h | $\beta(0.57)$, $\gamma(0.081)$, $\beta^+(0.653)$, $\gamma(0.511)$ | 2.5 mm |
| 89Sr | 50.5 d | $\beta(1.49)$ | 8.0 mm |
| 90Y | 2.67 d | $\beta(2.28)$ | 12.0 mm |
| 105Rh | 1.48 d | $\beta(0.57)$, $\gamma(0.320)$ | 1.9 mm |
| 109Pd | 13.6 h | $\beta(1.00)$ | 4.6 mm |
| 111Ag | 7.47 d | $\beta(1.05)$, $\gamma(0.340)$ | 4.8 mm |
| 153Sm | 1.95 d | $\beta(0.80)$, $\gamma(0.103)$ | 3.0 mm |
| 169Er | 9.50 d | $\beta(0.34)$ | 1.0 mm |
| 177Lu | 6.67 d | $\beta(0.50)$, $\gamma(0.208)$ | 1.5 mm |
| 111In | 67.4 h | $\gamma(0.173)$, $\gamma(0.247)$ | |
| 68Ga | 68 min | $\beta^+(1.90)$, $\gamma(0.511)$ | |
| 166Ho | 26.8 h | $\beta(1.85)$, $\gamma(0.081)$ | 8.7 mm |
| 198Au | 2.70 d | $\beta(0.97)$, $\gamma(0.411)$ | 4.4 mm |
| 213Bi | 1.00 h | $\alpha(7.80)$, $\gamma(0.720)$ | 70 $\mu$m |
| 72Zn | 46.5 h | $\beta(0.45)$ | 1.5 mm |
| 65Ni | 2.52 h | $\beta(2.14)$ | 10.0 mm |
| 165Dy | 2.3 h | $\beta(1.3)$ | 7.0 mm |

The chelating agent used to form the conjugate according to the invention is 2-(4-isothiocyanatobenzyl)-1,4,7,10-tetraazacyclododecane-1,4,7,10-tetraacetic acid, also known as p-SCN-Bn-DOTA or p-SCN-Bz-DOTA.

The process to obtain the conjugate according to the invention includes the steps of:

a) reacting human albumin (HA) with p-SCN-Bn-DOTA in an aqueous medium at a temperature between 35°C and 45°C, with the formation of a suspension, containing a solid phase suspended in an aqueous phase;

b) separating the solid phase, containing the conjugate, from the liquid phase, possibly containing unreacted p-SCN-Bn-DOTA;

Said separation step b) can be a filtration step or, preferably, an ultrafiltration step.

The solid obtained from the filtration step, as well as the retentate of the ultrafiltration step, can undergo freeze-drying. The conjugation procedure, object of this invention, preferably includes the use of extremely pure HA, hereinafter called HAP.

HAP is obtained from HA samples of European Pharmacopoeia quality, properly purified eliminating thermal stabilizers, as for example sodium caprylate and N-acetyl triptophan, added during the process of extraction and purification from the haemoderivative.

In order to promote the reaction between isothiocyanate and the nucleophilic sites of HA, including amine groups, these sites must be freed from thermal stabilizer molecules.

In fact, the interaction between the carboxylic group of tryptophan and the amine sites of HA is well-known, from the observations made by McMenamy et al. (JBC 1958, 233, 6, 1436-1446).

The purification is carried out by repeatedly washing the HA solution with 0.9% NaCl on ultrafiltration membranes having a cut-off of 10 kDa.

The method according to the present invention uses as a substrate mainly HA or human albumin obtained through the technology of recombinant DNA. Valuable substrates of this reaction can be water-soluble HA derivatives or HA analogues such as water-soluble proteins having composition, molecular weight and isoelectric point similar to HA, provided that they are highly purified.

HA has been used in therapy for numerous years and is thus present on the market with a purity regulated by numerous pharmacopoeias. For example, medicinal products based on HA in solution represent a source of high quality HA directly usable in humans. The conjugation reaction here described and the resulting reaction product, insoluble in aqueous environment, represent the present invention.

Medicinal solutions of HA contain variable amounts of thermal stabilizers, as in the case of solutions marketed by Baxter (Human Albumin Baxter), or by Alpha Therapeutic Italia SpA (Albutein) containing, in addition to 200 mg/ml of HA, 16 mmol/l (2.7 g/l) of sodium caprylate and 16 mmol/l (4.3 g/l) of sodium acetyl tryptophanate.

If HA containing a stabilizer is used, the first step of the process that leads to the insoluble conjugate according to the invention involves the purification of HA to remove, by using high ionic strength solutions, thermal stabilizers and other possibly present impurities. This purification phase is essential to allow to free the sites involved in conjugation with the isothiocyanate phenyl derivative of DOTA, then the reaction itself and its progress.

Such sites are represented by nucleophilic sites of the lateral chains of amino acids such as cysteine, lysine, histidine and arginine. Purification can be obtained by ultrafiltration or dialysis on membranes having a cut-off equal to 10 kDa in the presence of a solution of suitable ionic strength (0.1-0.5M, specifically 0.154M).

The conclusion of the purification process can be evaluated by UV spectrophotometry, by the absence of the typical absorbance of sodium acetyl tryptophanate ($\lambda$max =280 nm) in the ultrafiltrate of the last washing. GC-MS studies on the ultrafiltrate, dried and reconstituted with a suitable amount of acid methanol warmed for some minutes, show that when such absorbance is no more spectrophotometrically detectable, also the gas chromatogram of the related residual does not show any presence neither of methyl octanoate nor of N-acetyl tryptophan-methyl derivative.

The solution of purified HA (HAP) in 0.9% NaCl can be used as such or freeze-dried and used after reconstitution. The purification and freeze-drying processes allow to obtain HAP yields higher than 98% (w/w) with respect to the HA content of the medicinal solution used. The concentration of HA in HAP is determined by spectrophotometric UV dosing at 280 nm versus a pure standard HA (Albumin from human, fatty acid free, globulin free, A3782 Sigma-Aldrich). The HAP solution is stored between 2°C and 6°C and shows to be reactive for long to the conjugation with p-SCN-Bn-DOTA.

Such reaction occurs with a molar ratio of conjugation p-SCN-Bn-DOTA/HAP ranging between 100/1 and 1/1; in particular, in the range 16/1 to 30/1 yields of HAC product higher than 80% (wHAC/wHAP %) are obtained.

The conjugation reaction is influenced by different parameters including: pH, buffer species, buffering power, ionic strength, in addition to temperature and reaction time.

The molar ratio of reaction influences, in addition to the yield of the reaction, the degree of substitution (DS= nDOTA/nHA) of p-SCN-Bn-DOTA in the HAC conjugate.

"Degree of substitution" (DS) means the number of molecules of p-SCN-Bn-DOTA that react with reactive groups of HA (amine, oxidrilic and thiolic groups) per number of molecules of HA. This also corresponds to the number of DOTA structural moieties introduced in each molecule of HA.

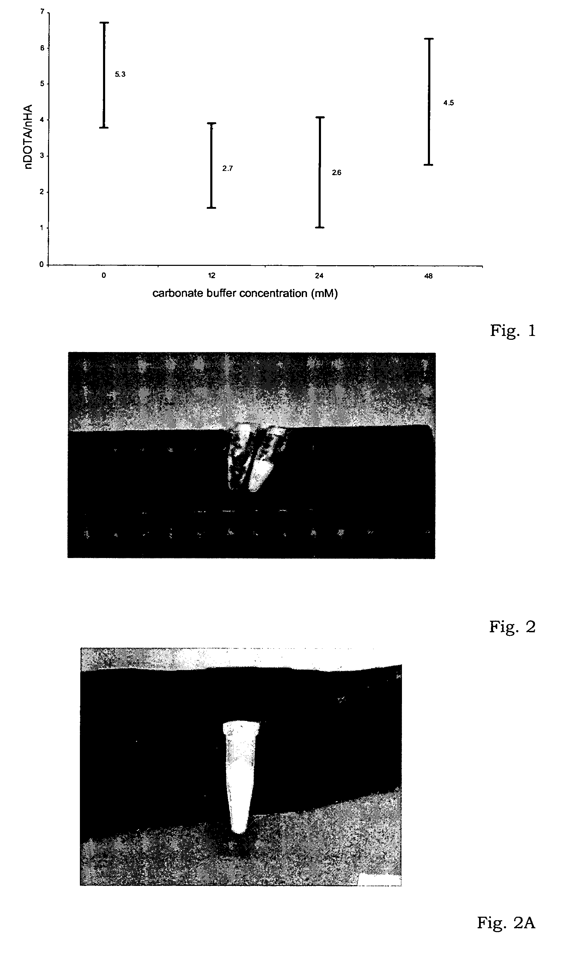

The reaction occurs between pH 4 and pH 8.5, and the pH of reaction influences the DS. In Fig. 1 the variation of the DS depending on the concentration of carbonate buffer in the reaction medium is reported. DS is expressed as a 95% confidence interval of the molar ratio given by the number of macrocyclic functions of p-SCN-Bn-DOTA added to one molecule of HA. The mean value is numerically indicated at the centre of the bar that represents the interval.

The presence or the absence of a buffer, such as for example a carbonate buffer or other efficient buffers in the above defined range of pH for the reaction, influences, also depending on the buffering power, the type of precipitation of HAC, which for this reason will appear in a more or less colloidal form.

Controlling the temperature of reaction is essential, in fact below 35°C the reaction does not proceed in a significant way. As optimal range of temperature for the reaction, the range between 35°C and 45°C, and more specifically the range $40 \pm 2$°C can be defined.

The reaction time influences the progress of the reaction and then the DS of the conjugate. After few minutes at 40°C the formation of a white gelatinous precipitate is appreciated, indicating the formation of the conjugate (Fig. 2A). A good yield of HAC is obtained prolonging the reaction time between 4 and 24 hours, depending on the reagents concentrations, pH, buffer system used, buffering power and ionic strength. An optimal time of reaction seems to be between 10 and 16 hours.

The formation of a gelatinous precipitate during the first hour generates high viscosity in the reaction medium that strongly restricts the diffusion of the reagent p-SCN-Bn-DOTA and then the progress of the reaction itself.

To this regard we have observed that it is appropriate to counter this increase of viscosity by dilution, to promote a good progress of the reaction and to obtain a suitable product for the subsequent radioisotopic labelling. To this aim, two aliquots of 0.9% NaCl solution, each equal to ¼ of the initial volume of the reaction medium, are added in two successive times, separated by 15 min, after the first hour of reaction.

The tactic of splitting the dilution in time allows to promote the diffusion of the reactive species without negatively influencing the reaction rate with a sudden decrease of p-SCN-Bn-DOTA concentration. The ponderal mean yield of HAC samples (n=5), purified, freeze-dried and with DS in the range between 2 and 5 (nDOTA/nHA), calculated in comparison with the weight of HAP used in the reaction, varies in the range 87% $\pm$ 5% (wHAC/wHAP %).

The product HAC is characterized by its insolubility in aqueous environment between pH 3 and pH 8.5.

In order to exclude that this precipitation is due to the denaturation and aggregation of HA in the reaction conditions, HAP was treated, in the presence of carbonate buffer, with DOTA, i.e. the chelating portion of the molecule without of the phenylisothiocyanate portion, at the concentrations used for the reaction.

After 20 h of treatment at 40°C no separation of solid phase (Fig. 2) from the initial solution was evident, this confirms that the insoluble product deriving from the reaction is a chemical entity different from HA.

From Fig. 2 the clear difference between the gelatinous precipitate obtained by the process according to the present invention (right microtube) and the solution deriving from the treatment of HAP with carbonate buffer and DOTA for 20 h at 40°C (left microtube) can be clearly inferred.

Fig. 2A documents the fact that, already in the first hour of reaction of the procedure according to the invention, the formation of a gelatinous precipitate occurs.

Such precipitate, properly purified by repeated washing with deionized water, recovered and freeze-dried, was subjected to IR analysis. The spectrum of such product results to be different from that of p-SCN-Bn-DOTA and from that of HAP.

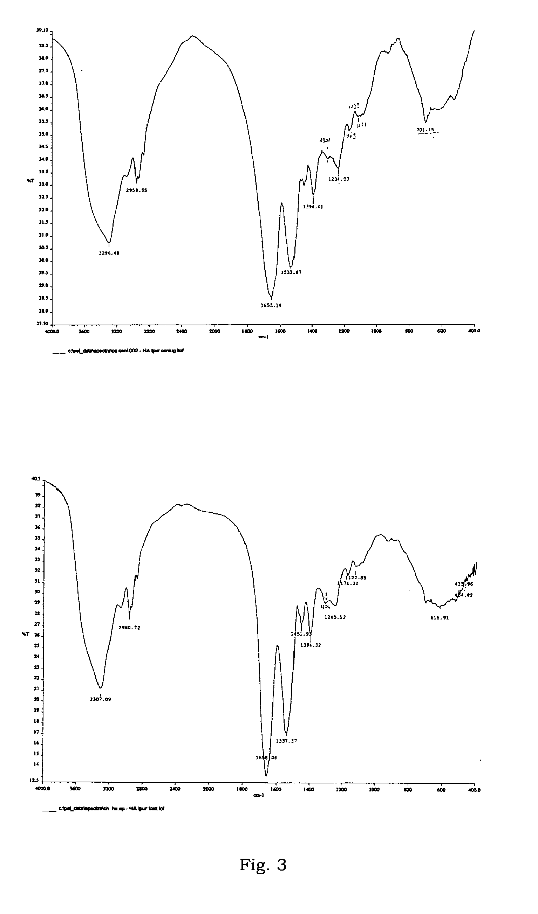

The comparison of the IR spectra in KBr of HAP (Figure 3, bottom) and of HAC (Figure 3, top), shows some spectral differences, attributable to the introduction of the C=S group, characteristic of the presence of a thioureic bond in the HAC conjugate, and described in the work of Ritchie et al. (A spectroscopic study of thiourea derivatives-(III): Infrared and Raman spectra of NN'-disubstituted thioureas Spectrochimica Acta Part A: Molecular Spectroscopy 27, 9, 1971, 1597-1608). Among these, the band enlargement around 1530 cm[-1] due to the presence of the C=S bond less polar than the C=O bond, the modifications in the spectral region between 1350-1200 cm[-1], but, mainly the absorbance at 700 cm[-1] characteristic of the C=S moiety indicating the formation of the thioureic bond responsible of the conjugation of DOTA with HA are noted. These spectral differences are reproducible and significantly relevant, although not particularly emphasized due to the not high degree of substitution of the small molecule of p-SCN-Bn-DOTA (MW 551.6) in the big molecule of HA (MW 66,500).

In Figure 3 a comparison between the IR spectrum of the freeze-dried HAC product object of the invention (top) and of the freeze-dried HAP purified albumin (bottom) subjected to the same reaction conditions, using DOTA instead of p-SCN-Bn-DOTA is shown.

The degree of substitution (DS) of HAC can be determined through a spectrophotometric UV methodology that utilizes the decrease of absorbance at 656 nm of the blue-coloured complex, between arsenazo III and Pb(II) due to the concurrent formation of a more stable complex between Pb(II) and DOTA.

In fact, DOTA forms kinetically inert complexes with Pb(II) with a high stability constant ($10^{23}$ -$10^{24}$) at room temperature (Dadachova E. et al. 1999. Spectrophotometric method for determination of bifunctional macrocyclic ligands in macrocyclic ligand-protein conjugates. Nuclear Medicine and Biology 26(8):977-982).

In the following Table 2 spectrophotometric dosing of the content of DOTA macrocycles per mole of HA is reported, expressed as DS $\pm$ CI$_{95}$ or as $\mu$moles DOTA/mg HAC $\pm$ CI$_{95}$, of some samples deriving from three microbatches (A, B, C) of HAC obtained at two different molar ratios of conjugation p-SCN-Bn-DOTA/HAP and related replicates.

Table 2

| Samples/ Replicates | Molar ratio of conjugation p-SCN-Bn-DOTA/HAP | DS nDOTA/nHA (mean $\pm$ CI$_{95\%}$) | $\mu$moles DOTA/mgHAC (mean $\pm$ CI$_{95\%}$) |
|---|---|---|---|
| A/1 | 20/1 | 3.01 $\pm$ 0.64 | 0.044 $\pm$ 0.009 |
| A/2 | 20/1 | 2.79 $\pm$ 0.53 | 0.041 $\pm$ 0.008 |
| B/1 | 20/1 | 3.06 $\pm$ 0.66 | 0.045 $\pm$ 0.009 |
| B/2 | 20/1 | 3.34 $\pm$ 0.68 | 0.049 $\pm$ 0.010 |
| C/1 | 23/1 | 5.92 $\pm$ 0.76 | 0.085 $\pm$ 0.010 |
| C/2 | 23/1 | 6.61 $\pm$ 0.79 | 0.094 $\pm$ 0.011 |
| C/3 | 23/1 | 5.99 $\pm$ 0.99 | 0.085 $\pm$ 0.013 |

Depending on the reaction conditions used, the conjugated $\mu$moles of p-SCN-Bn-DOTA per mg of HAC generally range from 0.02 (DS=1.3) to 0.2 (DS=15.0), even if the degree of substitution can be raised if needed, properly varying the reaction conditions, till getting near the maximum theoretical degree of substitution of 98, corresponding to reaction on all the nucleophilic sites present on HA: 1 -SH, 16 His, 58 Lys, 23 Arg, in which case the maximum number of conjugated $\mu$moles of p-SCN-Bn-DOTA per mg of HAC is equal to 0.813 and nDOTA/nHA=98.

In particular, samples containing from 0.03 to 0.15 $\mu$moles DOTA/mg HAC, corresponding to a DS ranging between 2 and 10 were studied through complexation with non-radioactive Lutetium ($^{175}$Lu) and with radioisotopes ($^{177}$Lu, $^{111}$In).

The complexation procedure with non-radioactive Lu allows to calculate the yield of incorporation of the metal in the macrocycle conjugated with HA, when this process is carried out at the same temperature (90°C) and time (30 min) conditions used for radioisotopic labelling. These samples were freeze-dried, following purification by ultrafiltration with deionized water from the excess of LuCl$_3$. The ponderal mean yield of the purified and freeze-dried complex HAC-Lu(III), calculated in comparison with the weight of HAC used, is equal to 100.0 $\pm$ 0.3 % (w/w) (CI$_{95}$, n=5).

HAC complexes with radionuclides can be brought to the required degree of purity for diagnostic and therapeutic applications, by using purification techniques known in the art.

By using molar ratios of Lu/HAC between 130/1 and 100/1 for complexation, yields of incorporation of Lu ranging between 40% and 60% are obtained, calculated on the basis of the moles of DOTA introduced per mg of HAC used, determined by a spectrophotometric method. Such yield, calculated using samples that contained not less than 2.4 moles DOTA/moles HA and not more than 4 moles DOTA/moles HA (Example 1) corresponded to a range of incorporation between 20 and 26 nmoles of Lu/ mg HAC (Example 2).

Also this measurement allows to indirectly establish, in addition to the efficiency of complexation in the labelling conditions, the conjugation of p-SCN-Bn-DOTA to HA. Furthermore, it confirms that the conjugation product HAC, although no more soluble in aqueous phase, is able to exert its typical metal complexing action, characteristic of DOTA, also in suspension, i.e. in a heterogeneous phase.

In the same way it was possible to demonstrate that HAC exerts its complexing action towards metals also after freeze-drying and reconstitution of the suspension.

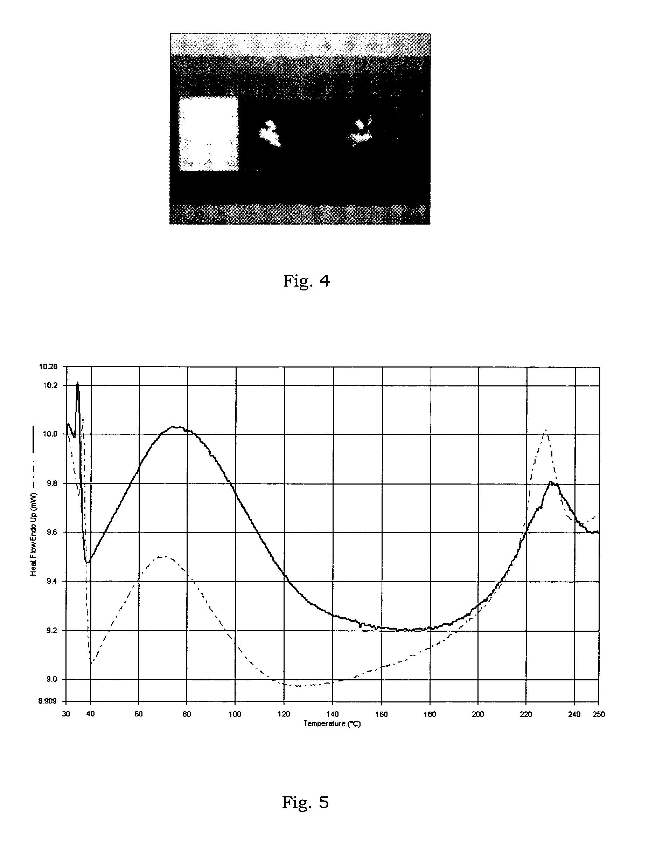

From Figure 4 one can observe that freeze-dried HAC is present in the state of powder (sample indicated with the C letter), while freeze-dried HAP (Fig. 4 on the left) is present with a markedly different aspect of flocky and light solid.

The product can be obtained in sterile form working in conditions of asepsis, starting from medicinal preparations of HA, thus certainly sterilized and apyrogen, and using buffers and reagents wherein sterility is obtained through sterilizing filtration through cellulose membranes with pore sizes ≤0.22 $\mu$m. For example, the addition of p-SCN-Bn-DOTA can be performed directly on concentrated or freeze-dried HA as a solution in 0.9% NaCl previously sterilized by filtration. All the other known procedures of sterilization by dry and humid heat in autoclave, by radiations, or other sterilization techniques known in the art can also be used.

Even if with lower yields and purity, the reaction of formation of the insoluble conjugate proceeds also in a sealed vial containing a solution of purified HA and the reagent p-SCN-Bn-DOTA in a suitable molar ratio, during a process of sterilization in autoclave with humid heat, i.e. in conditions of saturated steam.

The thermal behaviour of HAC during labelling is noteworthy. In fact, while HAP tends to coagulate when it is heated for 30 min at 90°C, HAC remains dispersed in suspension.

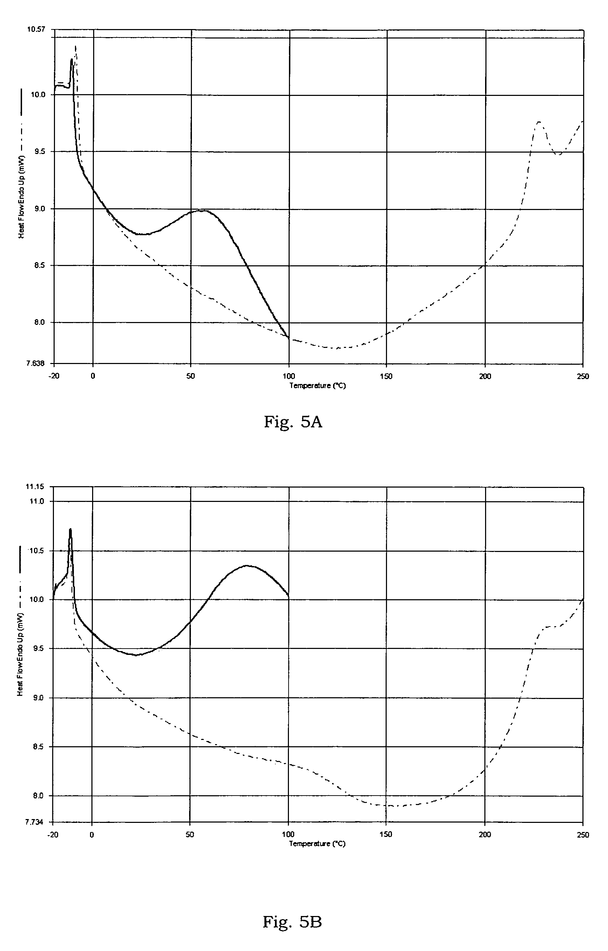

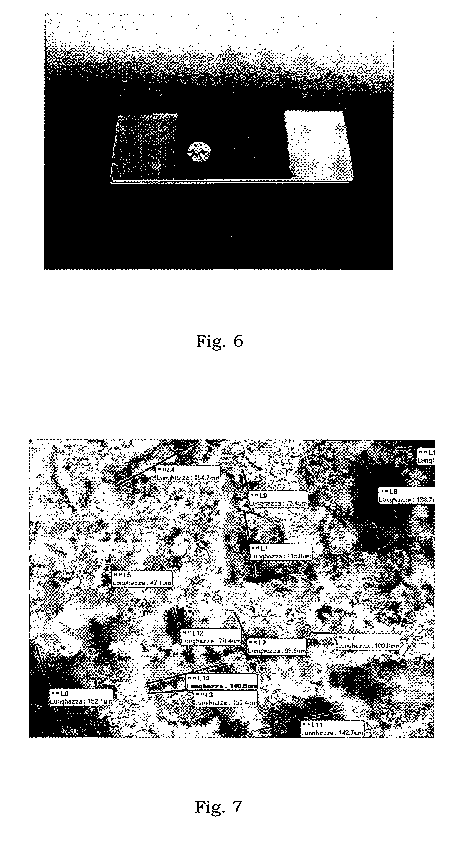

The different thermal behaviour is observed also in the comparison of the DSC profiles (see Fig. 5, 5A, 5B) and in the aspect of the samples after thermal treatment in DSC at 250°C: although both samples changed their colour due to baking, a different behaviour clearly appears: HAP forms a film (Fig. 6 on the left), while HAC remains in a powder state (Fig. 6 on the right).

The size distribution of the particles of conjugate is influenced by the reaction conditions. In mild conditions of temperature and for short times of treatment a conjugate is obtained that, after purification by repeated washing, is present in a suspension of sodium acetate as a colloidal precipitate having mean particle size larger than 10 $\mu$m. In prolonged reaction conditions this size tends to increase, remaining, anyway, smaller than 200 $\mu$m (Fig. 7). Such behaviour is observed also when the precipitate is subjected to different cycles of heating (40°C) and rapid cooling (5-6°C).

The control of the distribution of particle sizes of HAC in suspension can be carried out by centrifugation, in order to remove the particles with mean diameter smaller than 10 $\mu$m and by filtration on filters with controlled porosity in order to remove the particles with mean diameter larger than 100 $\mu$m. The selection of the distribution of particle sizes of HAC, obtained at the state of powder by freeze-drying, is feasible by sieving with certified sieves with minimum diameter of the lumen of the mesh of 20 $\mu$m and maximum diameter of the lumen of the mesh of 100 $\mu$m.

The redispersion of the freeze-dried product can be promoted with the aid of all the additives known to those who are skilled on the art, necessary to improve the stability of the freeze-dried product and its redispersion.

Preparation of a conjugate according to the invention

It is here described, by way of illustration and not of limitation, the preparation of a conjugate according to the present invention. Materials used and instruments used for said preparation are reported hereinafter.

MATERIALS

Ultrafiltration devices Amicon® Ultra-4 cut-off 10,000 NMWL (Millipore, USA); Dyalisis Cassettes Slide-A-Lyzer® 10K (Pierce, USA); Steril Apyrogenic syringe filters cellulose acetate Albet® Jacs-020-25, (Albet, Spain); 20% Albutein® 50 ml, (Alpha Therapeutic S.p.A, Italy); Human Albumin Baxter - 20 g/100 ml 50 ml bottle (Baxter S.p.A., Italy); Albumin from human, fatty acid free, globulin free, A3782 (Sigma-Aldrich, Germany); DOTA, M-140 (Macrocyclics, USA); p-SCN-Bn-DOTA, B-205 (Macrocyclics, USA); Ammonium acetate p.a. code 1,01116 (Merck, Germany); TraceCERT®, Lead standard for AAS, code 16595 (Sigma-Aldrich, Germany);

Arsenazo III p.a. code 11090 (Sigma-Aldrich, Germany); Sodium Acetate trihydrate p.a. code 1.06267 (Merck, Germany); Sodium Hydrogen Carbonate p.a. code 1.06329 (Merck, Germany); Sodium Chloride 0.9% saline (Eurospital, Italy); Sodium Chloride, p.a. code 1.06404 (Merck, Germany); Sodium Hydroxide solution (1.0N) code 5062-8576 (Agilent Technologies, Germany); Lutetium(III) Chloride code 450960 (Sigma-Aldrich, Germany); Hydrochloric Acid fuming 37% p.a. code 1.00314 (Merck, Germany); Acetic Acid Glacial 100% p.a. code 1.00063 (Merck, Germany); Lutetium ICP/DCP standard solution code 431818 (Sigma Aldrich, Germany), 65% $HNO_3$ trace analysis (Scharlau), ITLC-SG (Varian, USA), $^{177}LuCl_3$ (Perkin Elmer, USA), $^{111}InCl_3$ (Covidien, USA).

INSTRUMENTS

Freezone Lyophilizer 6 liter provided with stoppering tray dryer, LabConco, USA. DSC 7 Perkin Elmer, USA. UV-Vis Spectrophotometer HP 8453, Hewlett-Packard, USA. Orbital Mixing Dry Bath EchoThermTM SC25XT, Torrey-Pines Scientific, USA. Avanti J-30I High-Performance Centrifuge System, Beckman CoulterTM, USA. Microscope alphaphot-2 YS-2 Nikon, Japan provided with Moticam 2300 Motic, China. GC-MS EI HP5890(II)-HP5971A Hewlett-Packard, USA. FT-IR System 2000 Perkin Elmer, USA. The ITLC radiochromatographic profile was recorded by systems of autoradiography imaging with "high performance storage phosphor screen" (Cyclone, Packard BioScience, Meriden, CT, USA). Scintillation analyzer Packard USA. ICP-AES, J.Y. 24, Jobin-Yvon, France, provided with Cetac U-5000AT+ ultrasonic nebulizer, Cetac Technologies, USA. Bench steam sterilizer SteriPlus, De Lama, Italy, MilliQ water (Millipore, USA) or Purelab UHQ (Elga, UK).

Using the above materials and instruments, the preparation was carried out as follows.

0.5 ml of an aqueous solution containing 100 mg of human albumin (HA) are added to an ultrafiltration system with a regenerated cellulose membrane having a cut-off of 10 kDa (NMWL nominal molecular weight), hereinafter called UF10K, following normalization with 3.5 ml of 0.9% NaCl by ultrafiltration at 7400 g for 15 min.

0.9% NaCl is added until a final volume of 3.5 ml is reached and it is gently stirred by oscillation for 5 min in order to guarantee complete mixing of the two phases; ultrafiltration is carried out at 7400 g for 15 min.

The concentrated HA obtained after ultrafiltration, about 0.5 ml, is added with 0.9% NaCl to the final volume of 3.5 ml; after gentle stirring by oscillation for 5 min, in order to guarantee complete mixing of the two phases, centrifugation at 7400 g for 15 min is carried out. Such operation is repeated for at least other 3 times or until complete disappearance of the absorbance of N-acetyl triptophan in the ultrafiltrate, measured at 280 nm.

Purified human albumin (HAP) is resuspended with about 1.5 ml of 0.9% NaCl to obtain a 2 ml stock solution of HAP containing about 50 mg/ml of HA, which concentration is carefully determined by UV dosing at 280 nm versus a solution of pure standard HA, prepared dissolving 5.00 mg carefully weighted of pure standard HA free from fatty acids and globulins in 10 ml of 0.9% NaCl. The HAP stock solution is stored in the fridge or can be freeze-dried as such.

A 1.5 ml polypropylene tube is filled with 0.5 ml of HAP stock solution (or 25 mg of freeze-dried HAP reconstituted with deionized water) and there are added 5.00 to 6.00 mg of p-SCN-Bn-DOTA that are solubilised by stirring and possibly by sonication. A 60 $\mu$l aliquot of carbonate buffer at pH 9.0 in 0.9% NaCl, with a concentration ranging between 0.1M and 0.5M depending on the desired DS (see Fig.1), can be added to such solution.

The tube with the reaction mixture is subjected to heating at 40°C and orbital shaking at 300 rpm for 1 h on a thermoshaker. After such time a very viscous white gelatinous precipitate is formed, which marks the beginning of the conjugation reaction, at this point additional 250 $\mu$l of 0.9% NaCl are added and it is vortexed for 1 min. The sample is subjected to heating at 40°C and orbital shaking at 300 rpm for additional 15 min on a thermoshaker. At this point, additional 250 $\mu$l of 0.9% NaCl are added, it is vortexed for 1 min and then heating at 40°C under orbital shaking at 300 rpm on a thermoshaker is continued for 16 hours.

The raw product of the reaction, i.e. the suspension of conjugated albumin HA-p-SCN-Bn-DOTA (HAC) in the reaction medium, is transferred with the aid of 0.9% NaCl in an ultrafiltration device having a cut-off of 10 kDa; the volume of the suspension is brought to 3.5 ml with 0.9% NaCl and centrifugation at 7400 g for 15 min is carried out, following 5 min of gentle stirring by oscillation; such washing is repeated other two times bringing the volume of the concentrated retained by the ultrafiltration device to the volume of 3.5 ml with 0.9% NaCl. Then washing is performed always on the same ultrafiltration device with 1M sodium acetate buffer at pH 5, bringing the volume of the suspension to 3.5 ml and ultrafiltering by centrifugation at 7400 g for 15 min. The latter washing is repeated for at least 3 times or until complete disappearance of the UV absorbance spectrum of the macrocyclic reagent in the ultrafiltrate. The concentrated product, about 0.5 ml of suspension containing HAC in 1M sodium acetate buffer at pH 5, is transferred with the aid of the same buffer into a polypropylene tube of suitable capacity and brought to the final volume of 1.5 ml. Such suspension is stored in the fridge or freeze-dried.

SPECTROPHOTOMETRIC DETERMINATION OF THE DEGREE OF CONJUGATION OF p-SCN-Bn-DOTA WITH HA

A stock solution of Pb(II)-AA(III) (AA = arsenazo) in 0.15M AcONH$_4$, pH=7.00 is prepared, containing 67.62 $\mu$mol/l of Pb(II) and 140 $\mu$mol/l of AA(III). Such stock solution must be stored protected from light, at a temperature of 2-6°C and used at room temperature within one day after its preparation. A stock solution of DOTA 0.344mM in AcONH$_4$ (0.15M, pH=7.00) is prepared.

For the calibration curve, six standard solutions are prepared, each equal to a volume of 4.2 ml, containing increasing concentrations of DOTA from 0 to 0.041 mM.

3.4 ml of Pb(II)-AA(III) stock solution, 200 $\mu$l of 1M NaCl (in ammonium acetate) and a variable volume of DOTA stock solution (0-500 $\mu$l), depending on the desired final concentration, are added in each solution. The final volume of 4.2 ml for each standard solution is reached adding a suitable volume of AcONH$_4$ (0.15M, pH=7.00). For each solution, absorbance at 656 nm is determined, 10 min after its preparation at room temperature and protected from light. The reading is corrected subtracting the absorbance of a solution comprised of 4.0 ml of AcONH$_4$ buffer (0.15M, pH=7.00) and 200 $\mu$l of 1M NaCl.

The sample solution is prepared adding 0.90 to 1.10 mg of HAC into a solution containing 3.4 ml of Pb(II)-AA (III)stock solution, 200 $\mu$l of 1M NaCl, 600 $\mu$l of AcONH$_4$ buffer (0.15M, pH=7.00). Then absorbance at 656 nm is determined, 10 min after its preparation, at room temperature and protected from light. Results are expressed as $\mu$moles of DOTA/mg HAC or as DS = n moles DOTA/n moles HA.

DS is calculated through the following equation:

$$DS = \frac{MW_{HA}}{\dfrac{\mu gHAC}{\mu moles\ DOTA} - MW_{DOTANCS}}$$

where

$MW_{HA}$ = molecular weight of HA
$\mu gHAC$ = weighted micrograms of HAC freeze-dried sample
$\mu moles\ DOTA$ = micromoles of HA-conjugated DOTA calculated from the calibration curve
$MW_{DOTANCS}$ = molecular weight of p-SCN-Bn-DOTA

DIFFERENTIAL SCANNING CALORIMETRY (DSC)

Carefully weighted samples about 2.5 $\pm$ 0.5 mg were subjected to a thermal gradient of 10°C/min under a flow of nitrogen in an open aluminium melting-pot. Two types of scannings were carried out, from 30°C to 250°C and scannings from -20°C to 250°C with pre-heating from -20°C to 100°C.

FREEZE-DRYING PROCEDURE

The HAC sample, placed in a proper polypropylene tube, is frozen in a lyophilizer at -32°C. When thermal equilibrium is reached, as measured by a thermal probe immersed in the sample, a cold trap at -50°C is actuated and freeze-drying is started by generating vacuum. The process of freeze-drying, monitored by the thermal probe, for volumes of HAC suspension ranging from 1 to 10 ml, lasts between 16 to 24 hours. When the temperature of the product equals the temperature of the plate, the sample is subjected to secondary drying, at 30°C under vacuum, until reaching a constant weight of the freeze-dried product. The freeze-dried product is properly stored between 2 and 6°C protected from humidity.

Such procedure is also adopted to freeze-dry the HAP solution in 0.9% NaCl and to obtain a freeze-dried product to reconstitute to the original volume of suspension with sterile water, or to freeze-dry HAP samples following purification from NaCl by dialysis or ultrafiltration.

COMPLEXATION OF HAC WITH $^{175}$Lu, NON-RADIOACTIVE ISOTOPE.

8.00 mg of carefully weighted HAC are dispersed into 400 $\mu$l of 1M sodium acetate at pH 5 in a polypropylene tube. 100 $\mu$l of a solution containing 4.0 mg of $LuCl_3$ dissolved in HCl 0.05N are added to the dispersion. The tube is placed in a thermoshaker and the reaction is carried out at 90°C for 30 min under stirring at 400 rpm.

The cooled suspension is transferred into an ultrafiltration device and washed three times with 3 ml of 0.9% NaCl then with 3 ml of deionized water; such washings are sufficient to eliminate the excess of $LuCl_3$, given the absence of Lu, or its presence below of the limit of detection of the ICP-AES method, in the ultrafiltrate of the last washing. The aqueous suspension is then freeze-dried according to the procedure described above.

ICP-AES DETERMINATION OF $^{175}$Lu COMPLEXED WITH HAC (HAC-$^{175}$Lu)

All the determinations were performed at the wavelength of 261.542 nm by a calibration curve built with 6 aqueous solutions of increasing concentration containing up to 3 $\mu$g/ml of standard Lu.

The amount of $^{175}$Lu is determined in a carefully weighted HAC-Lu sample, in the range 0.90 to 1.10 mg. Such sample is dissolved in a polypropylene tube with 1 ml of 65% $HNO_3$ and kept at room temperature for 1 hour, before analysis the sample is diluted with 1 ml of UHQ water. The mean recovery of Lu in artificial samples (n=5) containing 1.0 mg of HAP is equal to 99.4 $\pm$ 0.2 % of $^{175}$Lu added therein.

HAC RADIOLABELLING

Radiolabelling is carried out with both $^{111}$In and $^{177}$Lu at the specific activity of 1 mCi/mg, using HAC (10-20 mg/ml) suspended in 1.0M sodium acetate pH 5.0.

A suitable volume of $^{177}LuCl_3$ or $^{111}InCl_3$ in 0.05N HCl is dosed in a sterile polypropylene tube already containing the HAC suspension in sodium acetate. After mixing it is heated at 90°C for 30 min.

RADIOCHEMICAL PURITY (RCP)

Radiochemical purity (RCP) is evaluated in triplicate for each radiolabelled sample by instant thin-layer chromatography (ITLC).

One aliquot of the radiolabelled suspension is mixed with 0.1 ml of a 2.5mM solution of diethylene-triamine-pentaacetic acid (DTPA) at pH 5.0. 5 $\mu$l of the radioactive suspension are deposed on an ITLC SG support and the development by ascending chromatography with 0.9% NaCl as mobile phase is carried out. In this chromatographic system, the insoluble HAC-radionuclide complex does not migrate, while the possibly present free radionuclide, bound to DTPA, migrates along with the solvent front. The radiochromatographic profile is determined by an autoradiographic system that uses a high performance storage phosphor screen (Cyclone, Packard BioScience, Meriden, CT, USA) and the radiochemical purity (RCP) is consequently calculated.

PROCEDURE FOR THE EVALUATION OF MICROBIOLOGICAL CONTAMINATION

The evaluation of microbic contamination, is carried out according to the guidelines for fill-tests, in an internal microbiology laboratory. Each vial containing 1.5 ml of HAC suspension in 1.0M sodium acetate at pH 5.0 is incubated in a thermostated oven (Heraeus Instruments series 6000) at (37 $\pm$ 1) °C for 14 days, and then qualitatively analyzed for the presence of any turbidity. In the case of a positive sample, 1 ml of this is plated on a Petri capsule to quantitatively evaluate the microbiological growth of the sample.

Example 1

SPECTROPHOTOMETRIC DETERMINATION OF THE DEGREE OF CONJUGATION OF p-SCN-Bn-DOTA WITH HA ON TWO HAC SAMPLES (BATCH 041010)

The procedure described in the previous section entitled "SPECTROPHOTOMETRIC DETERMINATION OF THE DEGREE OF CONJUGATION OF p-SCN-Bn-DOTA WITH HA" was carried out starting from a DOTA stock solution = 0.0178 mg/ml

$$R^2 = 0.9932 \quad S_{Yx} = 0.0175$$

Table 3

| Abs (Au) | mmoles of DOTA |
|---|---|
| 1.69 | 0.000 |
| 1.63 | 0.035 |
| 1.53 | 0.069 |
| 1.42 | 0.104 |
| 1.30 | 0.139 |
| 1.21 | 0.174 |

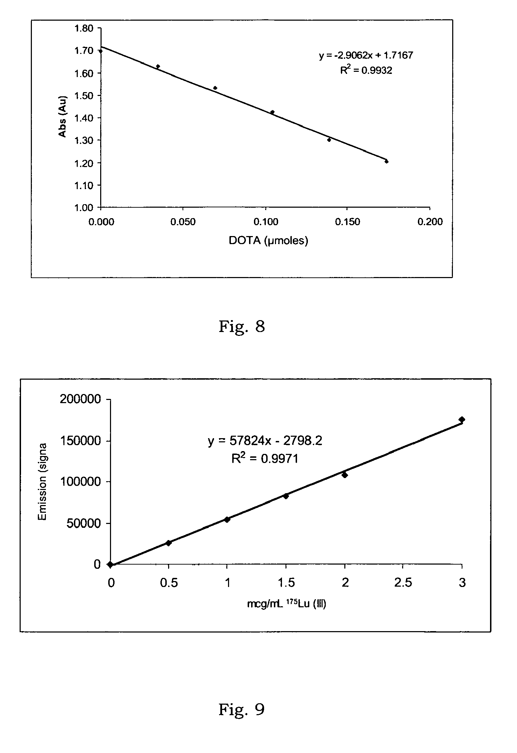

Based on the data reported in Table 3 the calibration curve of Figure 8 was built.
By virtue of the obtained calibration curve the values of degree of conjugation (DS) reported in the following Table 4 were calculated for the two samples.

Table 4

| Sample | HAC Batch | Absorbance (Au) | HAC (mg) | $\mu$moles of DOTA/mg HAC | DS (nDOTA/nHA) (95% confidence interval) |
|---|---|---|---|---|---|
| 1 | 041010 | 1.582 | 1.02 | 0.0454 | 2.40-3.72 |
| 2 | 041010 | 1.576 | 0.98 | 0.0493 | 2.66-4.02 |

Example 2

The procedure described in the previous section entitled "ICP-AES DETERMINATION OF $^{175}$Lu COMPLEXED WITH HAC (HAC-$^{175}$Lu)" was carried out on two HAC-$^{175}$Lu samples.
As explained in said section, first the calibration curve of Figure 9 was built, with six aqueous solutions of increasing concentration, containing up to 3 $\mu$g/ml of standard $^{175}$Lu, based on the values reported in the following Table 5.

$$R^2 = 0.9971 \quad S_{Yx} = 3776.93$$

Table 5

| μg/ml | Emission at 261.542 nm |
|---|---|
| 0 | 240 |
| 0.5 | 26123 |
| 1 | 53890 |
| 1.5 | 82767 |
| 2 | 107743 |
| 3 | 175043 |

By virtue of the obtained calibration curve, the values of the amounts (nmoles/mg of HAC-Lu) of complexed $^{175}$Lu reported in the following Table 6 were calculated.

Table 6

| Sample | HAC Batch | HAC-Lu | Emission (sample-matrix) | HAC-Lu (mg) | (nmoles Lu/mg HAC-Lu) (95% confidence interval) |
|---|---|---|---|---|---|
| 1 | 041010 | 051010 | 126203 | 1.16 | 20.8-23.3 |
| 2 | 041010 | 051010 | 118886 | 1 | 22.8-25.5 |

Example 3

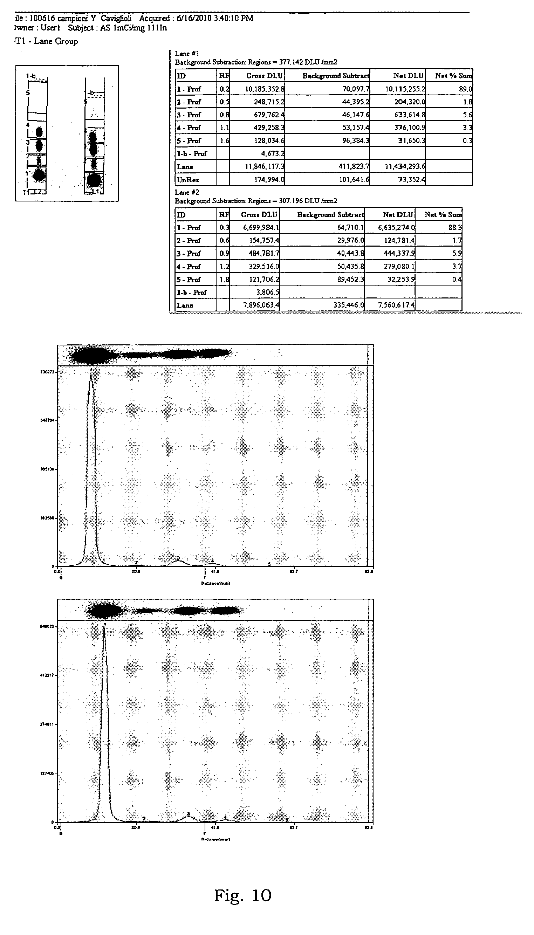

The procedure described in the previous section entitled "RADIOCHEMICAL PURITY (RCP)" was carried out on a HAC sample (batch 110610) labelled with [111]In.
Sample Y (Batch 110610): 16.7 mg/ml HAC in 1M sodium acetate pH 5. Specific activity of HAC labelled with $^{111}$In: 1 mCi/mg.
Results described in Figure 10 were obtained.

Example 4

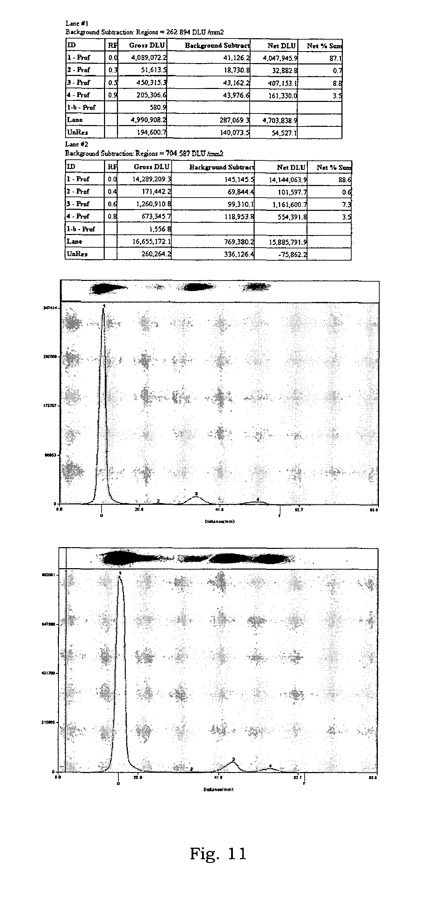

The procedure described in the previous section entitled "RADIOCHEMICAL PURITY (RCP)" was carried out on a HAC sample (batch 110610) labelled with [177]Lu.
Sample Y (Batch 110610): 16.7 mg/ ml HAC in 1 M sodium acetate pH 5.
Specific activity of HAC labelled with $^{177}$Lu: 1 mCi/mg
Results described in Figure 11 were obtained.

Example 5

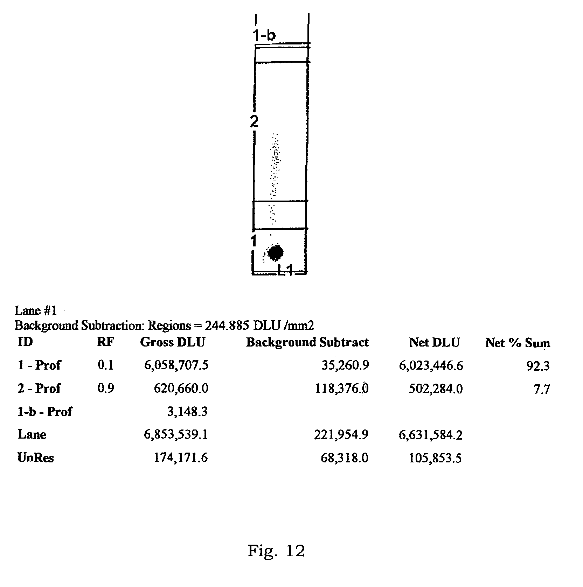

The procedure described in the previous section entitled "RADIOCHEMICAL PURITY (RCP)" was carried out on a HAC sample (batch 201109) labelled with [177]Lu.
Sample C (201109): 25 mg/ml HAC in 1M sodium acetate pH 5
Specific activity: $^{177}$Lu 10.2 Ci/mg
Specific activity of HAC labelled with $^{177}$Lu: 2 mCi/mg
Results described in Figure 12 were obtained.

Example 6

The stability of HAC labelled with $^{177}$Lu in saline, was evaluated measuring its RCP 6, 24, 48 and 144 hours after the labelling of HAC with [177]LuCl$_3$. Hereinafter all the experimental conditions of the stability test are reported and the values of %RCP are reported in the following Table 7.
HAC batch: Y110610
Date: 2010-06-30 Time: 10:00
Labelling
[177]LuCl$_3$

Preparation date 2010-06-22 Time 18:00
Specific activity $^{177}LuCl_3$: 20.37 Ci/mg
Volume:600 $\mu$l Concentration: 0.016 mCi/$\mu$l at calibration
Activity calculated at date 2010-06-30 and time 18:00 of calibration: 10 mCi
Activity calculated at date 2010-06-30 and time 10:00 of preparation: 10.2 mCi
Volume of suspension of HAC = 120 $\mu$l (corresponding to 2000 $\mu$g HAC) Specific activity of labelling: 1 mCi/mg
Incubation: 90°C for 30 min
Final volume: 240 $\mu$l Measured activity: 2 mCi (RCP control)

The residual volume from preparation by dilution of the samples of Example 7, was stored for 6 days and the RCP monitored according to the time intervals reported in the table. After 6 days, the sample showed the same RCP.

Table 7

| t (hours) | %RCP |
|-----------|------|
| 0 | 96 |
| 6 | 96 |
| 24 | 96 |
| 48 | 96 |
| 144 | 96 |

Example 7

STABILITY OF HAC LABELLED WITH $^{177}$Lu IN TISSUES

Two histopathological samples of spheroidal form, weight and diameter as reported in Table 8,

Table 8

| | Weight (mg) | Diameter (mm) |
|---|-------------|---------------|
| Sample A | 50.5 | 4.5 |
| Sample B | 73.6 | 5.1 |

derived from a surgical piece of mammalian carcinoma, containing internal tumoral proliferations, were inoculated with two doses of HAC-$^{177}$Lu, prepared as follows.
HAC batch: Y110610
Date: 2010-06-30 Time: 10:00
Labelling
$^{177}LuCl_3$
Preparation date 2010-06-22 Time 18:00 Specific activity $^{177}LuCl_3$: 20.37 Ci/mg
Volume: 600 $\mu$l Concentration: 0.016 mCi/$\mu$l at calibration
Activity calculated at date 2010-06-30 and time 18:00 of calibration: 10 mCi
Activity calculated at date 2010-06-30 and time 10:00 of preparation: 10.2 mCi
Volume of suspension of HAC = 120 $\mu$l (corresponding to 2000 $\mu$g HAC) Specific activity of labelling: 1 mCi/mg
Incubation: 90°C for 30 min
Final volume: 240 $\mu$l Measured activity: 2 mCi (RCP control)
Diluted 20 times
2 Doses of 100 $\mu$Ci/240 $\mu$l prepared

Said samples inoculated with HAC-$^{177}$Lu were stored in 5 ml of PBS (sterile phosphate buffered saline without $Ca^{++}$ and $Mg^{++}$) for 24 hours at 37°C.

After 24 hours the radioactivity of the two samples, measured by the probe, remained unchanged in comparison to the reading at the time of inoculation, while the gamma chamber did not detect any radioactivity in the storage solutions without inoculated samples.

Such samples stored at 2-6°C in PBS for additional 120 h showed to be as radioactive as at the time of inoculation while the presence of radioactivity was not detected in the storage solutions without inoculated samples for all the duration of the experiment.

**Claims**

1. A conjugate of human albumin or a water-soluble derivative or analogue thereof (HA) and 2-(4-isothiocyanatobenzyl)-1,4,7,10-tetraazacyclododecane-1,4,7,10-tetraacetic acid (p-SCN-Bn-DOTA), which is insoluble in aqueous medium at a pH of 3-8.5 and whose IR spectrum shows an absorption at about 700 cm$^{-1}$.

2. A process for preparing a conjugate of human albumin or a water-soluble derivative or analogue thereof (HA) and 2-(4-isothiocyanatobenzyl)-1,4,7,10-tetraazacyclododecane-1,4,7,10-tetraacetic acid (p-SCN-Bn-DOTA), comprising the steps of:

   a) reacting HA with p-SCN-Bn-DOTA in an aqueous medium at a temperature between 35°C and 45°C, thus forming a suspension, containing a solid phase suspended in a liquid phase;
   b) separating said solid phase, containing said conjugate, from said liquid phase, possibly containing unreacted p-SCN-Bn-DOTA.

3. The process according to claim 2, wherein said separation step b) consists of a filtration or ultrafiltration step.

4. The process according to claim 3, wherein a solid obtained from said filtration step or, respectively, a retentate obtained from said ultrafiltration step is subjected to freeze-drying.

5. The process according to any one of claims 2 to 4, wherein said reaction between HA and p-SCN-Bn-DOTA is carried out at a temperature of 38-42°C for 4-24 h, preferably 10-16 h.

6. The process according to any one of claims 2 to 5, wherein said aqueous medium is an aqueous solution with a ionic strength of 0.1-0.5M, in particular a solution of NaCl 0.9%, optionally buffered at pH 4-8.5.

7. The process according to any one of claims 3 to 6, wherein a cut-off of 10 kDa is used in said ultrafiltration step.

8. The process according to any one of claims 1 to 7, wherein the molar ratio between p-SCN-Bn-DOTA and HA is between 100:1 and 1:1, preferably between 30:1 and 16:1.

9. The process according to any one of claims 1 to 8, wherein the HA that is reacted with p-SCN-Bn-DOTA in said step a) has previously been purified by ultrafiltration or dialysis with a membrane having a cut-off of 10 kDa, in the presence of a solution with a ionic strength of 0.1-0.5M, in order to remove thermal stabilizers that are present in the commercially available HA, in particular sodium caprylate and sodium acetyl tryptophanate.

10. The conjugate of HA and p-SCN-Bn-DOTA of claim 1, obtainable by the process according to any one of claims 2 to 9.

11. A complex consisting of a conjugate of HA and p-SCN-Bn-DOTA (HAC) according to any one of claim 1 and 10 and a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu.

12. The complex according to claim 11, wherein said radioactive isotope is selected among the group consisting of $^{111}$In, $^{177}$Lu, $^{90}$Y, $^{67}$Cu and radioactive lanthanides and is preferably $^{177}$Lu.

13. The complex according to any one of claims 11 and 12 for therapeutic use.

14. The complex according to any one of claims 11 and 12 for use in the *in vivo* localization of radionuclides in organs and tissues of the human body for diagnostic or therapeutic purpose.

15. The complex according to any one of claims 11 and 12 for use in the localization of mammalian neoplastic lesions by the R.O.L.L. ("Radioguided Occult Lesion Localization") methodology.

16. A process for preparing an HAC complex according to any one of claims 11 and 12, comprising the steps of:

   - dispersing a conjugate according to claims 1 or 10 in an aqueous buffer at pH 4-6;
   - adding a water-soluble compound containing an ion of a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu, and
- heating under stirring at a temperature of 30-90°C for 20-40 minutes.

17. The process according to claim 16, wherein said water-soluble compound is $^{177}LuCl_3$.

18. The process according to any one of claims 16 or 17, wherein said aqueous buffer is an acetic acid/acetate buffer, preferably 1M with a pH of 5.

Fig. 1

Fig. 2

Fig. 2A

Fig. 3

Fig. 4

Fig. 5

Fig. 5A

Fig. 5B

Fig. 6

Fig. 7

Fig. 8

Fig. 9

ile : 100616 campioni Y Caviglioli   Acquired : 6/16/2010 3:40:10 PM
)wner : User1   Subject : AS 1mCi/mg 111In

T1 - Lane Group

Lane #1
Background Subtraction: Regions = 377.142 DLU /mm2

| ID | RF | Gross DLU | Background Subtract | Net DLU | Net % Sum |
|---|---|---|---|---|---|
| 1 - Prof | 0.2 | 10,185,352.8 | 70,097.7 | 10,115,255.2 | 89.0 |
| 2 - Prof | 0.5 | 248,715.2 | 44,395.2 | 204,320.0 | 1.8 |
| 3 - Prof | 0.8 | 679,762.4 | 46,147.6 | 633,614.8 | 5.6 |
| 4 - Prof | 1.1 | 429,258.3 | 53,157.4 | 376,100.9 | 3.3 |
| 5 - Prof | 1.6 | 128,034.6 | 96,384.3 | 31,650.3 | 0.3 |
| 1-b - Prof | | 4,673.2 | | | |
| Lane | | 11,846,117.3 | 411,823.7 | 11,434,293.6 | |
| UnRes | | 174,994.0 | 101,641.6 | 73,352.4 | |

Lane #2
Background Subtraction: Regions = 307.196 DLU /mm2

| ID | RF | Gross DLU | Background Subtract | Net DLU | Net % Sum |
|---|---|---|---|---|---|
| 1 - Prof | 0.3 | 6,699,984.1 | 64,710.1 | 6,635,274.0 | 88.3 |
| 2 - Prof | 0.6 | 154,757.4 | 29,976.0 | 124,781.4 | 1.7 |
| 3 - Prof | 0.9 | 484,781.7 | 40,443.8 | 444,337.9 | 5.9 |
| 4 - Prof | 1.2 | 329,516.0 | 50,435.8 | 279,080.1 | 3.7 |
| 5 - Prof | 1.8 | 121,706.2 | 89,452.3 | 32,253.9 | 0.4 |
| 1-b - Prof | | 3,806.5 | | | |
| Lane | | 7,896,063.4 | 335,446.0 | 7,560,617.4 | |

Fig. 10

Lane #1
Background Subtraction: Regions = 262.894 DLU /mm2

| ID | RF | Gross DLU | Background Subtract | Net DLU | Net % Sum |
|---|---|---|---|---|---|
| 1 - Prof | 0.0 | 4,089,072.2 | 41,126.2 | 4,047,945.9 | 87.1 |
| 2 - Prof | 0.3 | 51,613.5 | 18,730.8 | 32,882.8 | 0.7 |
| 3 - Prof | 0.5 | 450,315.3 | 43,162.2 | 407,153.1 | 8.8 |
| 4 - Prof | 0.9 | 205,306.6 | 43,976.6 | 161,330.0 | 3.5 |
| 1-b - Prof | | 580.9 | | | |
| Lane | | 4,990,908.2 | 287,069.3 | 4,703,838.9 | |
| UnRes | | 194,600.7 | 140,073.5 | 54,527.1 | |

Lane #2
Background Subtraction: Regions = 704.587 DLU /mm2

| ID | RF | Gross DLU | Background Subtract | Net DLU | Net % Sum |
|---|---|---|---|---|---|
| 1 - Prof | 0.0 | 14,289,209.3 | 145,145.5 | 14,144,063.9 | 88.6 |
| 2 - Prof | 0.4 | 171,442.2 | 69,844.4 | 101,597.7 | 0.6 |
| 3 - Prof | 0.6 | 1,260,910.8 | 99,310.1 | 1,161,600.7 | 7.3 |
| 4 - Prof | 0.8 | 673,345.7 | 118,953.8 | 554,391.8 | 3.5 |
| 1-b - Prof | | 1,556.8 | | | |
| Lane | | 16,655,172.1 | 769,380.2 | 15,885,791.9 | |
| UnRes | | 260,264.2 | 336,126.4 | -75,862.2 | |

Fig. 11

Lane #1

Background Subtraction: Regions = 244.885 DLU /mm2

| ID | RF | Gross DLU | Background Subtract | Net DLU | Net % Sum |
|---|---|---|---|---|---|
| 1 - Prof | 0.1 | 6,058,707.5 | 35,260.9 | 6,023,446.6 | 92.3 |
| 2 - Prof | 0.9 | 620,660.0 | 118,376.0 | 502,284.0 | 7.7 |
| 1-b - Prof | | 3,148.3 | | | |
| Lane | | 6,853,539.1 | 221,954.9 | 6,631,584.2 | |
| UnRes | | 174,171.6 | 68,318.0 | 105,853.5 | |

Fig. 12

Europäisches
Patentamt

European
Patent Office

Office européen
des brevets

**EUROPEAN SEARCH REPORT**

Application Number

EP 10 19 7071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FOWLER J CHARLOTTE ET AL: "Dual-isotope lymphoscintigraphy using albumin nanocolloid differentially labeled with In-111 and Tc-99m", ACTA ONCOLOGICA (STOCKHOLM), vol. 46, no. 1, 2007, pages 105-110, XP002631492, ISSN: 0284-186X | 1,10-14 | INV. A61K51/08 A61P35/00 |
| Y | * abstract * * page 106, column 1, paragraph 3 - column 2, paragraph 2 * ----- | 15 | |
| X,D | SCHILLER ET AL: "Yttrium-86-labelled human serum albumin microspheres: relation of surface structure with in vivo stability", NUCLEAR MEDICINE AND BIOLOGY, ELSEVIER, NY, US, vol. 35, no. 2, 20 December 2007 (2007-12-20), pages 227-232, XP022460571, ISSN: 0969-8051 | 1,10-14 | |
| | | | TECHNICAL FIELDS SEARCHED (IPC) |
| Y | * abstract * * page 229, column 1, paragraph 2 - paragraph 3 * ----- | 15 | A61K |
| X | US 2003/059368 A1 (GROMAN ERNEST V [US] ET AL) 27 March 2003 (2003-03-27) * example 50 * * paragraph [0236] * ----- | 1,10-13 | |

-/--

The present search report has been drawn up for all claims

| Place of search | Date of completion of the search | Examiner |
|---|---|---|
| The Hague | 6 April 2011 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 19 7071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | VAN DER PLOEG ET AL: "'Radioguided occult lesion localisation' (ROLL) for nonpalpable breast lesions: A review of the relevant literature", EUROPEAN JOURNAL OF SURGICAL ONCOLOGY, LONDON, GB, vol. 34, no. 1, 21 December 2007 (2007-12-21), pages 1-5, XP022392778, ISSN: 0748-7983 * the whole document * | 15 | |
| A,D | JAKUBOWSKI NORBERT ET AL: "Labelling of proteins with 2-(4-isothiocyanatobenzyl)-1,4,7,10-tetraazacyclododecane-1,4,7,10-tetraacetic acid and lanthanides and detection by ICP-MS", JOURNAL OF ANALYTICAL ATOMIC SPECTROMETRY, vol. 23, no. 11, 2008, pages 1497-1507, XP008135186, ISSN: 0267-9477 * abstract * * page 1499, column 2, paragraph 2 - page 1500, column 1, paragraph 2 * * table 1 * | 1-18 | |
| A,D | POVOSKI STEPHEN R ET AL: "A comprehensive overview of radioguided surgery using gamma detection probe technology", WORLD JOURNAL OF SURGICAL ONCOLOGY, BIOMED CENTRAL, LONDON, GB, vol. 7, no. 1, 27 January 2009 (2009-01-27), page 11, XP021050393, ISSN: 1477-7819, DOI: DOI:10.1186/1477-7819-7-11 * page 13, column 2, paragraph 3 - page 14, column 1, paragraph 1 * | 1-18 | |

TECHNICAL FIELDS
SEARCHED (IPC)

The present search report has been drawn up for all claims

| Place of search | Date of completion of the search | Examiner |
|---|---|---|
| The Hague | 6 April 2011 | Monami, Amélie |

EPO FORM 1503 03.82 (P04C01)

**EP 2 471 557 A1**

## ANNEX TO THE EUROPEAN SEARCH REPORT
## ON EUROPEAN PATENT APPLICATION NO.

EP 10 19 7071

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2011

| Patent document cited in search report | | Publication date | Patent family member(s) | Publication date |
|---|---|---|---|---|
| US 2003059368 | A1 | 27-03-2003 | US 2006067881 A1 | 30-03-2006 |

EPO FORM P0459

For more details about this annex : see Official Journal of the European Patent Office, No. 12/82

30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4024233 A
- US 4094965 A
- US 6730286 B

### Non-patent literature cited in the description

- **PAGANELLI G. ; LUINI A. ; VERONESI U. et al.** Use of technetium-99m-labeled colloid albumin for preoperative and intraoperative localization of nonpalpable breast lesions. *Journal of the American College of Surgeons,* 2000, vol. 190 (6), 692-699
- **PAGANELLI G. ; VERONESI U.** Innovation in early breast cancer surgery: radio-guided occult lesion localization and sentinel node biopsy. *Nucl Med Commun,* 2002, vol. 23 (7), 625-627
- **VAN DER PLOEG C. et al.** Radioguided occult lesion localisation' (ROLL) for nonpalpable breast lesions: A review of the relevant literature. *EJSO,* 2008, vol. 34 (1), 1-5
- **POVOSKI P. et al.** A comprehensive overview of radioguided surgery using gamma detection probe technology. *World J Surg Oncol,* 2009, 7
- **BOWEN et al.** Preparation of Technetium-99m Human Serum Albumin Macroaggregates. *Am J of Hospital Pharm.,* 1969, vol. 26, 529-534
- **WATANABE N. et al.** *Journal of Nuclear Medicine,* 1997, vol. 38 (10), 1590-1592
- **PATIL.** *Drug Dev. Res.,* 2003, vol. 58, 219-247
- **SCHILLER et al.** *Nuclear Medicine and Biology,* 2008, vol. 35 (2), 227-232
- **JAKUBOWSKI et al.** *J. Anal. At Spectrom.,* 2008, vol. 23 (11), 1497-1507
- **B. WESSELS ; R. ROGUS.** Radionuclide selection and model absorbed dose calculations for radiolabelled tumour associated antibodies. *Med Phys.,* 1984, vol. 11 (5), 638-645
- Metallic radionuclides for radioimmunotherapy. **A.R. FRITZBERG ; C.F. MEARES.** Cancer radioimmunotherapy: Present and Future. Harwood Academic Publishers, 1999, 57-79
- **MCMENAMY et al.** *JBC,* 1958, vol. 233 (6), 1436-1446
- **RITCHIE et al.** A spectroscopic study of thiourea derivatives-(III): Infrared and Raman spectra of NN'-disubstituted thioureas. *Spectrochimica Acta Part A: Molecular Spectroscopy,* 1971, vol. 27 (9), 1597-1608
- **DADACHOVA E. et al.** Spectrophotometric method for determination of bifunctional macrocyclic ligands in macrocyclic ligand-protein conjugates. *Nuclear Medicine and Biology,* 1999, vol. 26 (8), 977-982